# EUROPEAN PATENT SPECIFICATION

(11) **EP 0 845 269 B1**
(45) Date of publication and mention of the grant of the patent: **13.03.2002**
(21) Application number: 97120955.6
(22) Date of filing: 28.11.1997
(51) Int. Cl.: A61K 38/22, A61K 9/127

(54) **Liposomal human calcitonin gene-related peptide composition and preparation of the same**
Liposomale Zusammensetzung mit dem menschlichen Calcitonin Gene-Related Peptide und deren Herstellung
Composition lyposomale contenant le peptide apparenté au gène de calcitonine et sa préparation

(30) Priority: 29.11.1996 CN 96120902; 17.10.1997 CN 97119060
(43) Date of publication of application: 03.06.1998
(73) Proprietor: Beijing Richina Biotechnological Co., Ltd., Beijing 101101 (CN)
(72) Inventor: Wo, Weihan, 5412 Puch (AT)
(74) Representative: Olgemöller, Luitgard, Dr.

(56) References cited:
- EP-A- 0 335 597
- EP-A- 0 451 791
- WO-A-96/03993
- WO-A-97/06813
- SHEKHAR Y C ET AL: "Effects of prolonged infusion of human alpha calcitonin gene-related peptide on hemodynamics, renal blood flow and hormone levels in congestive heart failure" AM J CARDIOL, vol. 67, 1991, pages 732-736, XP002092912

## Description

### Background of The Invention

This invention relates to liposomal complex of human calcitonin gene-related peptide (liposomal hCGRP) composition and the preparation of the same, in particular to a product obtained by combining phospholipid and hCGRP .

Human α-type calcitonin gene-related peptide (hCGRP) is an endogenous neuromodulator and is known as the most potent vasodilator up to now. Its marktable product can be purchased in the world. However, hCGRP, as shown in other peptides, is unstable in storage (*in vitro,* aqueous solution) and circulation (*in vivo*) with half-life of 9-12 min, and it is difficult to utilize such a peptide as a drug for clinical application.

In order to shed a new light on hCGRP as a clinical drug, this object of the invention is to provide a liposomal hCGRP and the preparation of the same, whereby a novel phospholipids are associated with hCGRP to obtain a very stable and effective product. This liposomal hCGRP can release hCGRP gradually from the liposome to achieve long-term effect with half-life of 72 min *in vivo,* which can be effective in preventing and curing cardiovascular diseases.

### Description of The Invention

In order to realize this objective, the present invention relates to a pharmaceutical composition of hCGRP comprising liposomes prepared from natural soybean phospholipid. It is characterized by the fact that the weight ratio of hCGRP to soybean phospholipid is 1-2 to 100-8000(w/w), specially 1.5-2 to 2500-6000(w/w).

Said pharmaceutical composition of liposomal hCGRP containing 20-2000pg hCGRP in 5ml said composition is more preferred.

Mannitol, sorbitol, isotonic saline and dextrose or other pharmaceutical acceptable materials can be added in said pharmaceutical composition of liposomal hCGRP.

The present invention also relates to a method for preparing the pharmaceutical composition of the liposomal hCGRP characterized by following steps:
(1) adding sterilized and distilled water to purify and dry soybean phospholipid in a weight ratio of lipid to water being greater than 1 to 1000, followed by sonicating to obtain small and single-membrane vesicles of lipid bilayer;
(2) mixing hCGRP, dissolved in H₂O with a ratio of peptide to H₂O of 1 to 1000-25000, with the above soybean phospholipid in a ratio of the peptide to lipid being 1-2 to 100-8000, more particularly 1.5-2 to 2500-6000, sonificating and incubating at 37°C for 30-60 min to obtain a stable composition of liposomal hCGRP.

The composition of liposomal hCGRP thus obtained can be further lyophilized, and then dissolved in H₂O to obtain a aqueous solution containing 20-2000pg hCGRP per 5ml solution.

The present invention is of interest in a method for treating hypertension and congestive heart failure of a human by administrating to the patient the aforementioned pharmaceutical composition of liposomal hCGRP. Said method includes intravenous infusion, oral, nasal mucosal spray. Among them, intravenous dose of liposomal hCGRP is 0.1-10 pg hCGRP per kg body weigh. The bioavailability of liposomal hCGRP is approximately 80%.

Detail illustration is shown in the following.

### Theoretical basis of preparation of liposomal complex of hCGRP

Composition and sequence of the amino acids of hCGRP are characterized by (1) 8 of 37 amino acids of hCGRP are polar amino acids, with hydrophilic side chains, and 16 of 37 amino acids are apolar with hydrophobic side chains; (2) 4 of 8 polar amino acids are basic amino acids with positive charges in H₂O, and pH of hCGRP is 10. One molecule hCGRP contains 2 arginine, 2 lysine (Lys) and 1 aspartic acid (Asp). Arg and Lys are charged positively, Asp is charged negatively at physiological pH. hCGRP in which the ratio (ΣLys + ΣArg)/ (ΣGlu + ΣAsp)=4, is a very strong basic peptide. In physiological pH hCGRP is charged by net 3 positive charges. hCGRP contains 16 hydrophobic amino acids and 6 hydrophilic amino acids, and is a very typical amphoteric molecule.

By analysis of phospholipid composition, specially soybean phospholipid, it has been ascertained that (1) acidic lipids with negative charges in head groups in H₂O are greater than 40% of total phospholipid, (2)unsaturated fatty acid in soybean phosphglycerides are approx 70%, with protection effect from oxidation and hydrolyzation, (3) at the limit of very low lipid concentration (lipid:H₂O<1:100 w/w), the thermodynamically stable state is the dispersion of single walled vesicles of soybean phospholipid bilayers. The vesicle size range is 20-50 nm.

In the present invention by thin layer chromatography and gas-phase chromatography the soybean phospholipid components were analyzed, corrected and quantified using a standard phospholipids (Sigma). The soybean phospholipid used for clinical injection contains 44.9% acidic phospholipids including phosphatidylserine (17.2%), phosphatidylglycerol (8.1%), phosphatidylinositol (15.2%) and cardiolipin (4.4%) with rich negative charges in H₂O, and linoleic acid (58.31%), palmitic acid (24.36%), linolenic acid (7.32%), oleic acid (5.9%) and stearic acid (3.88%) with 71.53% unsaturated fatty acid and 28.47% saturated fatty acid. Liposome structure, as noted earlier, are formed spontaneously in H₂O by phospholipid molecules, from many different phospholipids, and the composition most frequently used has been the natural phospholipid extracted from cell membrane, such as soybean phospholipid [Imperial Chemical Industrial Ltd. and National Research Development Corporation, British Patent 15239651977]. Small single membrane liposomes range in diameter from approx. 200Å to 500Å, and consist of a single lipid bimolecular layer surrounding an aqueous compartment. Small single membrane liposome is characterized by (1) osmotic insensitivity (2) about 70% of the total lipid is located in the outer leaflet of the vesicle (3) by the very low lipid concentration limit with the thermodynamically stable state being the single membrane vesicles of lipid bilayer [Gruler H. Microstructure and transport properties of single shelled vesicles and monolayers of lipid mixtures and lipid/protein alloyes, in Liposomes Drugs and Immunocompetent Cell Functions. Edited by Claude Nicolau 1981.9915-27] (4) medium to large liposomes (MLV and LUV) are cleared rapidly from circulation after i.v. administration, while small unilamellar liposomes offer the potential for sustained drug release in blood stream and targeting to tissues other than the reticuloendothelial cell. Great emphasis is placed upon liposome as a biomembrane model, creating the possibility of *in vivo* application in medicine and research [Yang F.Y., The Application of Liposome in The Research in Biomembrane and Pharmacology, *SHENWUHUAXUE YU SHENGWUWULI JINZHAN* (*Biochemistry And Biophysics*, 1977 6:36]. Soybean phospholipid is a novel lipid existing in biomembrane, which have been used for the preparation of artificial membranes, such as liposomes [Biomembrane Group, Institute of Biophysics, Chinese Academy of Sciences, *SHENWUHUAXUE YU SHENGWUWULI JINZHAN* (*Biochemistry And Biophysics*, 1978 4:1].

By sonication and incubation the possibility presents itself for the polar interaction of the negatively charged group of the phospholipid with the positive charged groups of the amino acid residues of hCGRP on the outer surface of the membrane through ionic bonding with water. The apolar groups are located in the hydrophobic area of the membrane as a result of hydrophobic force, which includes the tails of the phospholipid and the hydrophobic amino acid residues from the hCGRP while the thermodynamic stability of liposomal hCGRP has been achieved, with an increase of half-life from 9-12 to 72 min and long-term storage in aqueous solution for two years (original storage time of 15 days). The effective dose of only 10⁻⁵ of liposomal hCGRP represents remarkable reduction in dosage and furthermore it can be absorbed by mucosal administration including oral, nasal and rectum mucosa with bioavailability of approximately 80%. Clinical studies indicated that remarkable treatment effect of liposomal hCGRP on 200 patients with hypertension and congestive heart failure had been achieved, and no secondary effect had been observed.

### Example 1

### Preparation of liposome from soybean phospholipid

25g soybean phospholipid was rotary evaporated (by using rotary evaporator, XZ-6, produced by Zhongkeyuan Kelong Corp.) from a chloroform:methanol (2:1,v/v) solution to form a thin golden film on the walls of a 1000 ml round bottomed flask.

After the last obvious traces of solvent had been removed, rotary evaporating was continued for 15 min, followed by drying for a further 15 min under a nitrogen atmosphere. The lipid was suspended in 250ml of distilled water and shaken with a few glass beads using a shaker (HZS-D, produced by Harbin Donglian Corp.) and then by an ultrasound bath machine (DF-6P3c, produced by Ningbo Xinyi Research Institute) for 30 min.

### Reconstitution of hCGRP in liposome membrane

10 mg hCGRP was dissolved in 250 ml destilled water, and stirred for 5min. hCGRP solution was mixed with the above liposome solution (250ml), stirred for 5 min, and sonicated for 2-3min, three times with interval of 3-5 min (by using an ultrasound bath machine, DF-6P3c, produced by Ningbo Xinyi Research Institute). Then the mixture was incubated at 37°C for 40 min (by using an ultrasound bath machine, produced by Harbin Donglian Corp.).

### Sedimentation of liposomal hCGRP

The reconstituted solution was sedimented by Ultracentrifuge (400000×g, for 40min, at 4°C, VAC 602, WEB Leipzig, Germany) and washed with distilled water three times.

### Lyophilization and Resolution

The sedimented liposomal hCGRP was lyophilized by using lyophilizer LGJ (produced by Instrument Plant of Academy of Military Medical Sciences (China)) and dissolved in distilled water (passed through 6# sterilizing filter) (lipid:H₂O=1:1000w/w), the above solution was sterilized at 100°C for 30 min, and enclosed.

### Example 2

The procedure of Example 1 was repeated using various ratios of peptide to lipid(w/w). hCGRP reconstitution in membrane, the reconstitution efficiency and the stability of the final product were compared, as shown in Table 1.

**Table 1.**

| Effect of Liposomal hCGRP with various ratios of peptide to lipid(w/w) in preparative procedure for the reconstitution efficiency and vasodilatory activity. | | |
|---|---|---|
| Ratio of hCGRP to lipid (w/w) | Free hCGRP (%) | Relative Vasodilatory Activity (%) |
| 1:1 | 80.2±10.1% | 0.02±0.01% |
| 1:10 | 24.5±3.6% | 1.2±0.3% |
| 1:100 | 15.7±1.9% | 32.6±6.7% |
| 1:1000 | 0.1±0.02% | 95.1±11.4% |
| 1:10000 | 0.1±0.03% | 94.9±13.9% |
| 1:250000000 | 0±0% | 100±0% |

Method: liposomal hCGRP was prepared according to the procedure of Example 1 with various ratios of peptide to lipid (w/w), followed by centrifugating and determining of hCGRP content in supernatant liquid as free hCGRP not reconstituted in the membrane. After that each group of samples were divided into two groups, one was stored in -70°C condition after being sterilized and sealed as control, and another was dissolved in H₂O with the ratio of lipid to H₂O being 1 to 1000(w/w) stored at room temperature after sterilized and stored under nitrogen. After storage time of 24 months vasodilatory activity of samples was measured, shown as % of control. Each date is mean±SD of five independent data.

In Table 1 it is seen that when the ratio of lipid to peptide was above 1000(w/w), hCGRP had been reconstituted in membrane with little free hCGRP, and vasodilatory activity remained at above 95% after 24-month-storage.

### Example 3

In the following test, a similar procedure to that of Example 1 was repeated with the exception of using various ionic strength to study the effect of ionic strength in solution on reconstitution efficiency of hCGRP in lipid membrane. Table 2 list the effect of ionic strength on hCGRP reconstitution.

**Table 2:**

| the effect of ionic strength in solution on reconstitution efficiency of hCGRP in lipid membrane. | | | | | |
|---|---|---|---|---|---|
| NaCl Conc. (mM) | 0 | 10 | 50 | 100 | 150 |
| Free hCGRP | 0.2±0.1% | 11.6±1.8% | 24.5±4.2% | 35.5±7.9% | 36.1±4.1% |

Method: In an NaCl aqueous solution with different concentrations, hCGRP was reconstituted in soybean phospholipid membrane, followed by centrifugating and determination of free hCGRP content in supernatant liquid, shown as % of total hCGRP. The ratio of peptide to lipid was 1 to 1000(w/w), each date was mean ± SD of five independent experimental data.

In Table 2, it is indicated that reconstitution efficiency decreased with the increasing ionic strength of the solution. The purified H₂O solution may offer a favorable environment for interaction and association between lipid and peptide.

### Comparative Example 1

The same procedure of Example 1 was repeated with the exception of using two non-charged lipids instead of soybean phospholipid.

By determination of free hCGRP content in the supernatant liquid after centrifugation and chromatographic analysis, it was shown that the reconstitution efficiency of hCGRP in soybean phospholipid membrane achieved 99.9%, but in PC and PE membrane were only 21.2% and 30.3% respectively, indicating that negative charge of phospholipid is very important for reconstituting hCGRP in membrane successfully.

**Table 3.**

| Comparison of reconstitution efficiency of hCGRP in soybean phospholipid (SP), phosphatidylcholine (PC), and phosphatidylethanolamine (PE) membrane(%). | | | | |
|---|---|---|---|---|
| Sample | Kd | % | Kd | % |
| hCGRP | 0.52 | 100% | 0.04 | 0% |
| hCGRP+PC | 0.52 | 79.8% | 0.04 | 21.2% |
| hCGRP+PE | 0.52 | 69.3% | 0.04 | 30.3% |
| hCGRP+SP | 0.52 | 0.1% | 0.04 | 99.9% |

### Comparative Example 2

Three different phospholipid including soybean phospholipid (SP), phosphatidylcholine (PC) and phosphatidylethanolamin (PE) were used for liposomal hCGRP preparation, and the structure integrity of hCGRP in three liposome membranes was analyzed during 24-month storage by HPLC, as shown in Table 4.

**Table 4.**

| Effect of various phospholipids on integrity of hCGRP reconstituted in lipid membrane | | | | | | | | | |
|---|---|---|---|---|---|---|---|---|---|
| | time (months) | | | | | | | | |
| | 0 | 3 | 6 | 9 | 12 | 15 | 18 | 21 | 24 |
| SP(% area) | 98.4 | 98.4 | 99.0 | 98.0 | 97.8 | 97.8 | 97.1 | 97.1 | 97.0 |
| | ±2.4 | ±2.1 | ±1.9 | ±2.4 | ±2.1 | ±2.0 | ±1.8 | ±1.9 | ±1.7 |
| PC(% area) | 99.2 | 90.2 | 83.3 | 76.4 | 70.1 | 68.2 | 66.1 | 65.8 | 65.1 |
| | ±3.3 | ±2.9 | ±3.4 | ±3.1 | ±2.6 | ±2.5 | ±1.9 | ±2.0 | ±1.9 |
| PE(% area) | 98.9 | 94.5 | 89.2 | 85.3 | 82.1 | 76.6 | 70.3 | 68.5 | 64.4 |
| | ±2.9 | ±3.1 | ±2.2 | ±3.3 | ±1.9 | ±1.7 | ±1.6 | ±2.1 | ±2.2 |

Method: hCGRP in sample was extracted with acid solution and analyzed by reverse phase HPLC; retention time and peak area of hCGRP during chromatography was recorded. Each data is mean ±SD of five independent experimental data. hCGRP standard (BACHEM, Swizerland) was used for correction of retention time.

Results in Table 4 indicated that little change of hCGRP purity was observed during 24-month storage with decrease of 1.4%, indicating that hCGRP renconstituted in soybean phospholipid membrane is very stable during storage, but in PE and PC membranes are unstable and integrity of hCGRP remained only 64.4% and 65.1%.

### Comparative Example 3

In the following test, phosphatidylcholine (PC) and phosphatidylethanolamin(PE) were used for liposome-hCGRP preparation and compared with soybean phospholipid liposomal hCGRP. During 24-month storage vasodilatory activities of three various liposomal hCGRP were determined and compared as shown in Table 5.

**Table 5.**

| Effect of various phospholipid on vasodilation of hCGRP reconstituted in lipid membrane (ED₅₀ values ×10⁻⁸ mg/ml) | | | | | | | | | |
|---|---|---|---|---|---|---|---|---|---|
| Samples | storage (months) | | | | | | | | |
| | 0 | 3 | 6 | 9 | 12 | 15 | 18 | 21 | 24 |
| Control (at -70°C, n=5) | | | | | | | | | |
| mean | 4.7 | 4.9 | 5.2 | 5.1 | 5.3 | 5.2 | 5.1 | 5.2 | 5.3 |
| ± SD | ±0.6 | ±0.5 | ±0.7 | ±0.8 | ±0.5 | ±0.7 | ±0.6 | ±0.6 | ±0.7 |

| PS in H₂O (at 25°C, n=5) | | | | | | | | | |
|---|---|---|---|---|---|---|---|---|---|
| mean | 5.1 | 4.9 | 4.9 | 5.3 | 5.5 | 5.2 | 5.5 | 5.6 | 5.9 |
| ± SD | ±0.7 | ±0.8 | ±0.9 | ±0.7 | ±0.6 | ±0.7 | ±0.8 | ±0.6 | ±0.8 |

| PC in H₂O (at 25°C, n=5) | | | | | | | | | |
|---|---|---|---|---|---|---|---|---|---|
| mean | 4.9 | 15.2 | 50.6 | 151 | 451 | 865 | 1133 | 1566 | 1923 |
| ± SD | ±0.8 | ±3.2 | ±10.3 | ±21.2 | ±99.3 | ±153 | ±333 | ±439 | ±544 |

| PE in H₂O (at 25°C, n=5) | | | | | | | | | |
|---|---|---|---|---|---|---|---|---|---|
| mean | 5.2 | 10.3 | 33.4 | 99.1 | 329 | 634 | 903 | 1234 | 1633 |
| ± SD | ±0.7 | ±2.4 | ±6.5 | ±15.8 | ±82.8 | ±101 | ±125 | ±289 | ±345 |

Methods: Experiments were performed on New Zealand white rabbits ( weight 2.5-3.5kg) that were anesthetized with pentobarbital sodium (30mg/kg, i.v.).

Rabbits were placed in headholer, diameters of ocular vessels (conjuctive) were measured by use of a microscope equipped with a TV camera coupled to a video monitor. Images were recorded in computer and vessel diameters were measured later with an image analyzer software. The analytical system of microcirculation was purchased from DAHENG Co. China. 10 µl of diluted samples was dropped into the eye of the rabbits and the images of ocular vessels were recorded in the computer.

During storage of 24 months, ED₅₀ values (×10⁻¹⁸ mg/ml) for vasodilation of liposomal hCGRP were determined. Little changes in soybean phospholipid membrane of liposomal hCGPR were observed. But, for PC and PE liposomal hCGRP, their ED₅₀ decreased 100-1000 folds after 24-month storage, indicating negative charges in lipid membrane is very important for stable association between the peptide with lipid.

### Experiment 1

### Analysis Of hCGRP -liposome complex

Methods: Liposomal hCGRP and free hCGRP were analyzed by the method of Berk [Berk D. and Marcinka K., Gel Chromatography in Separation Methods. Deylz ed. 1984, 271]. hCGRP samples reconstituted with or without soybean phospholipid (1.0 ml in 0.1M Tris-HCl, pH8.8) were applied to a 1.5×46cm sephadex G-50 fine column in 0.1M Tris-HCl, pH8.8. Blue dextran 2000 (Pharmacia) and ³²PO₄ (England) were mixed with separated sample as mark of Vo and Vi respectively.

Free hCGRP in Sephadex G-50 fine was chromatographed at Kd=0.52, but liposomal hCGRP Kd=0.44, similar to blue dextron 2000, indicating that the liposome-hCGRP complex had been formed. The data obtained by gel filtration were described in Table 5.

**Table 6.**

| Determination of hCGRP binding with soybean phospholipid | | | |
|---|---|---|---|
| | Kd (G-50) | | |
| hCGRP | 0.52 | 0.54 | 0.51 |
| hCGRP + Lipid | 0.04 | 0.04 | 0.05 |
| Blue Dextran 2000 | 0 | | |
| ³²PO₄ | 1 | | |

Each data entry represents individual experiment result.

As shown in Table 6, after the reconstitution of hCGRP with soybean phospholipid, much larger complex of hCGRP with lipid than hCGRP had been formed, indicating that the properties of hCGRP and soybean phospholipid give a new light on the preparation of a stable liposomal hCGRP.

### Experiment 2

### Analysis of physical and chemical stability of liposomal hCGRP

It is clear that any liposomal formulation must have adequate stability over the time period between its preparation and ultimate use so as to be a pharmaceutical carrier. The surface of liposome membrane, as mentioned above, has large amounts of negative charges, which prevents change of their size induced via the fusion between liposomes. In an environment of large amounts of water, the negative charged particles are in a thermodynamic stable state and large amount of unsaturated tails in phospholipid can reduce possibility of water molecule inserting themselves into lipid bilayer and thus preventing degradation of lipid and peptide molecules from auto-hydrolysis and auto-oxidation.

Phospholipids are subject to hydrolysis in aqueous media, resulting initially in the formation of the corresponding lysophospholipid and fatty acid. During the storage of our liposome or liposomal hCGRP, the contents of lyophospholipid were determinated as a criteria of chemical stability by TLC, while the size of liposome was measured by gel filtration observing the position of elution peak as a criteria of physical stability [Szoka F., et al., Comparative properties and methods of preparation of lipid vesicles (liposomes), Ann. Rev. Biophys. Bioeng. 1980 9:467.5].

### Analysis LPC Content

Changes of LPC content in soybean phospholipid liposome. Storage conditions: at 25°C, samples in H₂O Lipid : H₂O=1:1000(w/w) and sterilized at 100°C for 30 min and sealed.

Methods: Analysis of LPC content in the liposome was done by TLC. Silica gel H (Type 60) from E. Merk in Germany. LPC standard was purchased from Sigma, as control of LPC in samples, and its Rf value is 0.04 in our experimental conditions. After samples were sterilized at 100°C for 30min, the chemical stability of liposome membrane was determined by analysis of LPC content at interval of 3 months during storage. Each data is mean ±SD of 5 independent TLCs. The mixture of the samples and standard LPC was used for corrective assay by single direction TLC and double direction TLC, indicating that LPC of the mixture was only one component on the silica gel.

During storage period of 24 months, the content of LPC was increased progressively from 2.1±0.34% to 4.7±0.51% (p<0.01) for soybean phospholipid vesicles and from 1.9±0.22% to 3.4±0.46% (p<0.01) for the liposomal hCGRP, respectively. The degradation percentages of phospholipid molecules were 2.6% and 1.5% for liposome and liposomal hCGRP respectively, indicating that the reconstituted hCGRP can increase stability of membrane by its positively charged groups which interact with negatively charged groups of phospholipid.

Determination of liposome size was carried out by means of gel filtration (Kd) during storage period. Kd values were unchanged either for soybean phospholipid liposome or for the reconstituted liposome with hCGRP, indicating that our liposomes are thermodynamically stable in 1000:1(H₂O: phospholipid, w/w) environment during 24 months storage after sterilization.

### Experiment 3

### Analysis of the stabilities of hCGRP reconstituted in liposome membrane

Stability of hCGRP reconstituted in liposome membrane was observed by (A) Sephadex G-50 fine gel filtration to measure the dissociation of hCGRP from the liposome; (B) microcirculation observation system to see the vasodilatory activity, and compare with free hCGRP during storage period.

### A. The stability of association of hCGRP with membrane (Table 8).

Methods: During storage period of the reconstituted liposome after being sterilized at 100°C for 30 min, free hCGRP, dissociated from the liposome, was determined by gel filtration at the interval of 3 months. The absorption at UV 206 run of hCGRP has been correlated with 0.52 of the distribution coefficient (Kd) in correction of hCGRP standard. At this Kd value, we can investigate whether hCGRP is dissociated from the reconstituted liposome during storage period. Sephadex G-50 fine gel filtration was carried out, with each data being mean±SD of 3 independent operations.

During the storage period of 24 months the hCGRP reconstituted in the liposome membrane was not dissociated into free hCGRP by observation of the 206nm absorption band of eluent solution at Kd=0.52. This result has shown us that hCGRP can form very stable complex with soybean phospholipid vesicles by our experimental procedure based on the characteristics of their molecular structure. All samples were stored at 25°C after sterilized and sealed. The ratio of lipid: H₂O is 1:1000 in the reconstituted liposome of soybean phospholipid.

### B. Measurement of vasodilatory activities of hCGRP

hCGRP is an endogenous neuromodulator and most powerful vasodilatator known by us. We investigated vasodilatory activities of hCGRP reconstituted in the liposome membrane of soybean phospholipid and in comparison with free hCGRP in H₂O and human plasma during storage period.

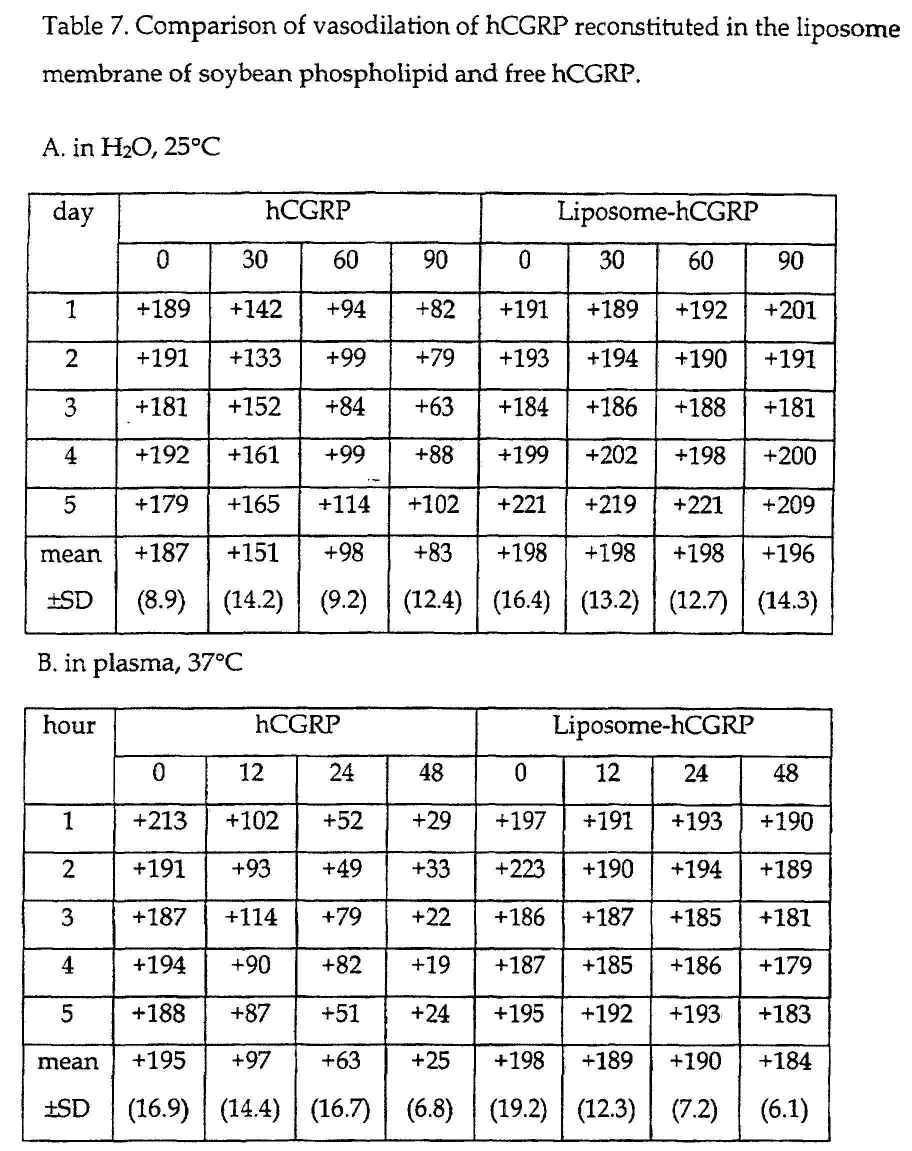

Methods: Experiments were performed on New Zealand white rabbits (weight 2.5-3.5 kg) that were anesthetized with pentobarbital sodium (30mg/kg, i.v.). Rabbits were placed in a headholer, diameters of ocular vessels (conjuctive) were measured by use of a microscope equipped with a TV camera coupled a video monitor. Images were recorded in computer and vessel diameters were measured later with an image analyzer software. The analytical system of microcirculation was purchased from DAHENG Co. China. 10µl of diluted samples was dropped into the eye of the rabbits and the images of ocular vessels were recorded in a computer. The vessel diameter of the images was analyzed by the microcirculation software.

In H₂O, the vasodilatory activities (% diameter) of free hCGRP and hCGRP reconstituted in liposome membrane of soybean phospholipid were altered from +187±8.9% to +83±12.4% (p<0.001, n=5) and from +198±16.4 to +196±14.3% respectively after a storage period of 90 days. In human plasma, their activities decreased from +195±16.49% to +25±6.8% (p<0.001, n=5) and from +198±19.2%to +184±6.1% respectively after incubation for 48 hours. These results indicated that the reconstituted hCGRP in the liposome membrane is more stable in comparison with free hCGRP.

### Treatment Experiment 1

### Role of liposomal hCGRP in treatment of patients with congestive heart failure (CHF)

Patients: The human studies were carried out in sixteen patients admitted for the control of congestive heart failure: seven were male and nine female, with an average age of 66.3 years (range 54 to 75 ). Six in New York Heart Association (NYHA) phase IV, seven in phase III and three in phase II [ Bruce R. A. Mod. Concepts Cardiovasc. Dis. 1956, 25:321-326]. All patients were treated with liposomal hCGRP after stopping treatment with other drug such as digoxin for three days.
Drugs: Liposomal hCGRP, prepared by Example 1, was used for treatment of the patients, containing 20 pg hCGRP / 5ml solution. Drug content is 2000BU/5ml.

### Dose administration route:

Mucosal absorption: via oral-nasal mucosal 40-80 BU (1-3 drops) three times per day; via anus2000BU 3 times per day;
Intravenous infusion: 2000-8000BU (2-4 ampoules) of Liposomal hCGRP added to 5% GS or 100-250ml 0.9% NaCl solution. 1 time per day.
Measurement: Before and after taking the drug, breathing rate, vesicular sound, heart rate and rhythm, liver size, swelling index, weight, urinary volume, and cardiac performance by means of ECG, Echocardiography were observed and measured daily.
Result: Liposomal-hCGRP had sustained beneficial effects on patients with CHF. Most patients felt symptomatically better the next morning. There was a dominant cardiac improvement in 9 patients, effective in 6, and only one remained unchanged. No subject complained of side-effect to the drug such as headache, flushing. The drug did not cause hypotension and did not affect the liver or renal function during treatment.

**Table 8.**

| Treatment effect of liposomal hCGRP on patients with congestive heart failure | | | | | | |
|---|---|---|---|---|---|---|
| Patient No. | Pre-drug | OD | Days | Efficiency | | |
| | | | | DE | E | ND |
| 1. CO intoxication | III | i.n | 1 | | II | |
| 2. PCD | IV | i.n | 1 | | III | |
| 3. CO intoxication | III | i.n | 1 | | II | |
| 4. CAD | II | i.n | 7 | | | II |
| 5. HCD | II | i.n | 7 | | I | |
| 6. SCD | IV | i.v | 7 | I | | |
| 7. MI | IV | i.v | 7 | | II | |
| 8. MD | III | i.v | 7 | I | | |
| 9. MD | III | i.v | 7 | I | | |
| 10. MD | IV | i.v | 7 | | III | |
| 11. MD | III | i.v | 7 | I | | |
| 12. HCD | III | i.v | 7 | I | | |
| 13. HCD | IV | i.v | 7 | II | | |
| 14. HCD | IV | i.v | 7 | | II | |
| 15. HCD | IV | i.v | 7 | II | | |
| 16. HCD | III | i.v | 7 | I | | |
| * i.n: nasal mucosal administration; i.v: intravenous administration; PCD: Pulmonary cardiac disease; CAD: Coronary artery disease; HCD: Hypertensive cardiac disease; MI: Myocardial infarction; MP: Myocardial disease; DE: Dominant efficiency; E: Efficiency; ND: No difference. | | | | | | |

Discussion: Congestive heart failure (CHF) is usually caused by reduced cardiac output as a result of impaired myocardial contractivity, improvement of which is an important object of treatment in patients with CHF. Calcitonin gene related peptide (CGRP) is a neuropeptide with potent vasodilation and positive chronotropic and inotropic action on the heart, indicating that it may be used for CHF treatment. The recent studies have proved that intravenous infusion of CGRP (8.0ng/kg/min) for 8h caused a decrease in right arterial, pulmonary artery, pulmonary artery wedge and systemic arterial pressure.

Cardiac output, stroke volume, and renal blood flow and glomerular filtration increased. Application of liposomal hCGRP in this invention to 16 CHF patients gave beneficial effects, and was characterized by the following:
(1) hCGRP releases gradually from liposomes with long-term effect, have an average effective time of 10 hours, which is 5 fold greater than the results obtained in other investigations;
(2) easy absorption via mucosa, such as oral, nasal and anus administration;
(3) bioavailability of liposomal hCGRP being 10 fold greater than that of hCGRP reported in other investigations.

### Treatment Experiment 2

### Role of liposomal hCGRP in treatment of patients with essential hypertension

### Materials and Method:

Patients: Twenty one patients were hospitalized with essential hypertension of which ten male and eleven female, average age of 62.2 years (rang 45 to 73 ) and one had aldosteronism. Their hypertension ranged from 3 to 37 years, and their clinical diagnoses based on clinical information was clear. According to WHO/ISH 1993 hypertension diagnosis standard (Beijing Renmin Weigheng Chubanshe 1996, 227-228), patients in phase hypertension were 11, in III hypertension 10.
Drugs and Measurement: Sixteen patients stopped administration of other hypotensive drugs for two weeks, five patients with little hypotension were treated with mepramidil and carvedilol. Liposomal hCGRP, prepared in Example 1, was used for all patients by means of i.v infusion or oral-nasal mucosal administration.
   a. Oral-nasal mucosal: 0.05-0.10 ml liposome-hCGRP, containing 0.2-0.4 pg hCGRP, was given three times per day and for five consecutive days.
   b. Intravenous infusion: one ampoule of liposome-hCGRP, containing 20 pg hCGRP in 5 ml aqueous solution, was given in 100-500 ml 0.9% NaCl per day and for five preceding days.
   c. Measurement: Artery blood pressure (BP) was measured at 15, 30, 60, 120, 180 min after administration on the first day. In the following days, the BP were recorded 6 times before and after drug administration.

### Determination of hypertension-relieving:

According the diagnosis standard of 1979 cardiovascular epidemiology (Henan, Zhen Zhou, China) [J. Chinses Cardiovascular Diseases 1979 7:(2):18], hypotension of liposomal hCGRP was determined.
Dominant efficacy: diastolic pressure decrease > 100 mmHg to normotensive level, or only > 20 mmHg.
Efficacy: diastolic pressure decrease 10 mmHg and to normotensive level, or 10-19 mmHg.
Uneffective: diastolic pressure did not decrease to normotensive level or decrease < 10 mmHg.
   For patients only with systolic pressure increasing, hypotension of drug was determined according to the above standard, but systolic pressure decreasing should be more than 20 mmHg.
Results: 21 patients were treated with liposomal hCGRP prepared by the present invention, 4 mucosal administration, 4 intravenous infusion, 13 were given combinative administration of i.v. with mucosal, 2 via anius mucosa, 2 via oral mucosa, and rest via nasal mucosa.
Treatment result: Systolic pressure decreased 20-105 mmHg with an average decrease of 17 mmHg (p<0.001, n=21).
   Diastolic pressure was decreased 5-25 mmHg with an average decrease of 17 mmHg (p<0.001, n=21). Liposomal hCGRP was dominant efficacy for 13 patients, effective for 7 and ineffective for 1. Hypertension-relieving began within 5 minutes after administration, and was maintained approx. 10h.

### Secondary effect:

2 patients with chronic nasitis felt a little comfortless by nasal mucosa administration, after i.v. infusion was used, nasal symptom vanished. During treatment with liposomal hCGRP, headache and flushing did not occur, no liver and renal lesions was observed.

### Discussion

1. CGRP is endogenous neuropeptide. Liposomal hCGRP in the present invention has avoided rapid degradation of CGRP to achieve long-term effect by gradually releasing hCGRP in vivo, and it is easily absorbed by tissue cells. For treatment of hypertension, it takes effect very quickly, effectively and safely. In 21 patients dominant efficacy achieved 61.9%, total efficiency 95.2%, only one was ineffective, and no remarkable difference was observed for treatment efficiency of hypertension by either i.v. infusion or mucosal absorption of liposomal hCGRP.
2. Some reports indicated dose-dependent effect of CGRP on hypertension in animal with hypotension efficacy increased with increase in dosage. However, in 21 patients with hypertension in the experiment, optimal hypertension-relieving efficacy was observed by 40-80 BU liposomal hCGRP via nasal mucosal absorption, but further increase of the dose did not give a better result, which may be caused by increase of cardiac output induced by hCGRP positive inotropic action on heart.
3. Shekgar et al. [Shekhar YC, et al., Effects of Prolonged Infusion of Human Alpha Calcitonin Gene-Related Peptide on Hemodynamics, Renal Blood Folw and Hormone Levels in Congestive Heart Failure, Am J Cardiol 1991; 67:733.] reported that i.v. infusion of hCGRP (8.0ng/kg/min) for 8h with total dose of 3840ng/kg induced hypotension, systemic artery blood pressure decreased 18% (p<0.05) 30 min after drug intake. In this experiment, the hypotensive dose was 8000 BU/day, and hCGRP content was only 780 pg, i.e., 0.8 pg hCGRP per kg body weight, equal to 2.0×10⁻⁷ fold of the hCGRP dose reported by other investigations.
4. A small secondary effect was noted when 1 patients felt nasal comfortless after nasal administration, possibly related to nasal vasodilation. Thus patients with nasal disease should be treated by other route of administration of liposomal hCGRP.

## Claims

1. A pharmaceutical composition of hCGRP, comprising liposomes obtained from natural soybean phospholipid, in which the weight ratio of hCGRP to soybean phospholipid is 1-2 to 100-8000.

2. A pharmaceutical composition of hCGRP as claimed in claim 1, wherein the weight ratio of hCGRP to soybean phospholipid is 1.5-2 to 2500-6000.

3. A pharmaceutical composition of hCGRP as claimed in claim 1 or 2, containing 20-2000pg hCGRP in 5ml of said composition.

4. A method for preparing the pharmaceutical composition of hCGRP as claimed in claim 1, **characterized by** the following steps:
(1) adding sterilized and distilled water to purified and dried soybean phospholipid in a weight ratio of lipid to water being greater than 1 to 1000, followed by sonicating to obtain small and single-membrane vesicles of lipid bilayer;
(2) mixing hCGRP, dissolved in H₂O with the ratio of peptide to H₂O being 1 to 1000-25000, with the above soybean phospholipid in a ratio the peptide to lipid being 1-2 to 100-8000, more particularly 1.5-2 to 2500-6000, sonicating and incubating at 37°C for 30-60 min to obtain a stable composition of liposomal hCGRP.

5. A method as in claim 4, **characterized by** a weight ratio of hCGRP to soybean phospholipid of 1.5-2 to 2500-6000 in step (2).

6. A method as claimed in claim 4 or 5, **characterized by** the further step of lyophilizing, and then dissolving the liposomal hCGRP in H₂O to obtain an aqueous solution containing 20-2000pg hCGRP per 5ml solution.

7. A pharmaceutical composition as claimed in any of claims 1 to 3 for use in the treatment of hypertension of humans.

8. A pharmaceutical composition as claimed in any of claims 1 to 3 for use in the treatment of congestive heart failure of humans.

9. A pharmaceutical composition as claimed in claim 7 or 8, wherein it is to be administered to the patient by intravenous infusion, oral, nasal mucosal spray.

10. A pharmaceutical composition as claimed in any of claims 7 to 9, wherein it is to be administered to the patient in an amount of 0.1-10 pg hCGRP per kg body weight.

## Patentansprüche

1. Pharmazeutische hCGRP-Zusammensetzung mit Liposomen, die aus natürlichem Sojabohnen-Phospholipid gewonnen wurden und in denen das Gewichtsverhältnis von hCGRP zu Sojabohnen-Phospholipid 1-2 zu 100-8000 ist.

2. Pharmazeutische hCGRP-Zusammensetzung nach Anspruch 1, worin das Gewichtsverhältnis von hCGRP zu Sojabohnen-Phospholipid 1,5-2 zu 2500-6000 ist.

3. Pharmazeutische hCGRP-Zusammensetzung nach Anspruch 1 oder 2 mit einem Gehalt von 20-2000 pg hCGRP in 5 ml der Zusammensetzung.

4. Verfahren zum Herstellen einer pharmazeutischen hCGRP-Zusammensetzung nach Anspruch 1, **gekennzeichnet durch** die folgenden Schritte:
(1) Zusetzen von sterilisiertem und destilliertem Wasser zu gereinigtem und getrocknetem Sojabohnen-Phospholipid in einem Gewichtsverhältnis von Lipid zu Wasser, das größer als 1 zu 1000 ist, und anschließende Schallbehandlung zur Gewinnung von kleinen Einzelmembran-Vesikeln aus einer Lipiddoppelschicht;
(2) Vermischen von in H₂O gelöstem hCGRP, wobei das Verhältnis von Peptid zu H₂O 1 zu 1000-25000 beträgt, mit dem obigen Sojabohnen-Phospholipid, im Verhältnis von Peptid zu Lipid von 1-2 zu 100-8000, insbesondere 1,5-2 zu 2500-6000, Beschallen und 30-60minütiges Inkubieren bei 37°C, um eine stabile liposomale hCGRP-Zusammensetzung zu erhalten.

5. Verfahren nach Anspruch 4, **gekennzeichnet durch** ein Gewichtsverhältnis von hCGRP zu Sojabohnen-Phospholipid von 1,5-2 zu 2500-6000 in Schritt (2).

6. Verfahren nach Anspruch 4 oder 5, **gekennzeichnet durch** den weiteren Schritt einer Lyophilisierung und dann Auflösung des liposomalen hCGRP in H₂O, um eine wäßrige Lösung mit 20-2000 pg hCGRP pro 5 ml Lösung zu erhalten.

7. Pharmazeutische Zusammensetzung nach einem der Ansprüche 1 bis 3 zur Verwendung bei der Behandlung von Bluthochdruck beim Menschen.

8. Pharmazeutische Zusammensetzung nach einem der Ansprüche 1 bis 3 zur Verwendung bei der Behandlung von kongestiver Herzinsuffizienz beim Menschen.

9. Pharmazeutische Zusammensetzung nach Anspruch 7 oder 8, worin diese dem Patienten durch intravenöse Infusion, oral oder als Spray für die Nasenschleimhaut verabreicht werden soll.

10. Pharmazeutische Zusammensetzung nach einem der Ansprüche 7 bis 9, worin diese dem Patienten in einer Menge von 0,1-10 pg hCGRP pro kg Körpergewicht verabreicht werden soll.

## Revendications

1. Composition pharmaceutique de hCGRP, comprenant des liposomes obtenus à partir du phospholipide de soja naturel, dans laquelle le rapport pondéral du hCGRP au phospholipide de soja est un rapport de 1-2 à 100-8000.

2. Composition pharmaceutique de hCGRP suivant la revendication 1, dans laquelle le rapport pondéral du hCGRP au phospholipide de soja est un rapport de 1,5-2 à 2500-6000.

3. Composition pharmaceutique de hCGRP suivant la revendication 1 ou 2, contenant 20 à 2000 pg de hCGRP dans 5 ml de ladite composition.

4. Procédé pour la préparation de la composition pharmaceutique de hCGRP suivant la revendication 1, **caractérisé par** les étapes suivantes :
(1) addition d'eau stérilisée et distillée à du phospholipide de soja purifié et déshydraté en un rapport pondéral du lipide à l'eau de plus de 1 à 1000, avec ensuite un traitement par ultrasons pour obtenir de petites vésicules monomembranaires d'une double couche lipidique ;
(2) mélange de hCGRP, dissous dans H₂O, le rapport du peptide à H₂O étant un rapport de 1 à 1000-25 000, au phospholipide de soja précité en un rapport du peptide au lipide de 1-2 à 100-8000, plus particulièrement de 1,5-2 à 2500-6000, traitement par ultrasons et mise en incubation à 37°C-pendant un temps de 30 à 60 minutes pour obtenir une composition stable de hCGRP liposomal.

5. Procédé suivant la revendication 4, **caractérisé par** un rapport pondéral du hCGRP au phospholipide de soja de 1,5-2 à 2500-6000 dans l'étape (2).

6. Procédé suivant la revendication 4 ou 5, **caractérisé par** l'étape supplémentaire de lyophilisation, puis de dissolution du hCGRP liposomal dans H₂O pour obtenir une solution aqueuse contenant 20 à 2000 pg de hCGRP pour 5 ml de solution.

7. Composition pharmaceutique suivant Tune quelconque des revendications 1 à 3, destinée à être utilisée dans le traitement de l'hypertension chez l'homme.

8. Composition pharmaceutique suivant Tune quelconque des revendications 1 à 3, destinée à être utilisée dans le traitement de l'insuffisance cardiaque congestive chez l'homme.

9. Composition pharmaceutique suivant la revendication 7 ou 8, destinée à être administrée au patient par perfusion intraveineuse, buccale ou pulvérisation à la muqueuse nasale.

10. Composition pharmaceutique suivant l'une quelconque des revendications 7 à 9, destinée à être administrée au patient en une quantité de 0,1 à 10 pg de hCGRP par kg de poids corporel.
